# EUROPEAN PATENT APPLICATION

(11) **EP 0 933 375 A1**
(43) Date of publication of application: **04.08.1999**
(21) Application number: 97310212.2
(22) Date of filing: 17.12.1997
(51) Int. Cl.: C07D 487/04, G03C 1/73

(54) **Photochromic compounds**

(71) Applicant: Amersham Life Science Ltd, Amersham, Buckhinghamshire HP7 9LL (GB); UNIVERSITY COLLEGE CARDIFF CONSULTANTS LTD., Cardiff CF1 3TE (GB)
(72) Inventor: Heller, Harry George, Vale of Glamorgan, Wales, CF71 7RT (GB); Wenlock, Mark Carl, Shrewsbury, Shropshire, SY2 5PG (GB)
(74) Representative: Rollins, Anthony John

(57) **Abstract**

Fulgimide derivatives are provided having the formula: 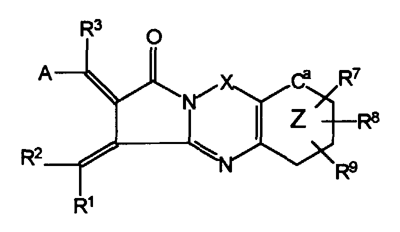 wherein groups R⁷, R⁸ and R⁹ are hydrogen or are chosen to provide desired solubility, reactivity and spectral properties to the compound;
X is a group -(CH₂)ₙ- or a group, 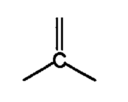 in which case it links via a two or three atom chain with carbon atom C^{a} of ring Z to form a fused bicyclic aromatic ring which may be optionally substituted and n is 0 or 1;
Z is an optionally substituted six-membered aromatic or fused bicyclic aromatic ring containing carbon atoms and optionally no more than two atoms selected from oxygen, nitrogen and sulphur.

The compounds are useful for imparting photochromic properties to materials by covalent and non-covalent association.

## Description

The present invention relates to a group of compounds which exhibit photochromism, their preparation and use as optical switches for luminescent materials. In particular, the invention relates to fulgimide derivatives.

Photochromism can be defined as the property of a material able to change reversibly its visible absorption spectrum upon exposure to activating radiation and to revert to its original absorption spectrum thermally on removal of the activating radiation, or on substitution of radiation of a different wavelength.

US Patent No.4220708 describes a series of photochromic succinic anhydride and succinimide derivatives (fulgides and fulgimides respectively) having the following general formula (1): 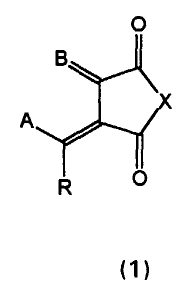
wherein X represents O, NR⁶, R⁶ being H, alkyl, aryl or an aralkyl group;
R represents and alkyl, aryl, aralkyl, or heterocyclic group;
A represents a 3-furyl, 3-thienyl, 3-benzofuryl or 3-benzothienyl group;
B represents a cycloalkylidene group or the group, 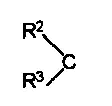 in which R² and R³ independently represent an alkyl, aryl, aralkyl or a heterocyclic group, or one of R² and R³ represents hydrogen and the other represents an alkyl, aryl, aralkyl, or heterocyclic group.

Compounds of this class undergo cyclisation when exposed to ultraviolet radiation, ring closure taking place between the carbon atom to which groups R² and R³ are attached and the 2-position of the furyl or thienyl ring. The photocyclisation reaction for fulgide ring structures is illustrated by the following scheme (Scheme 1).

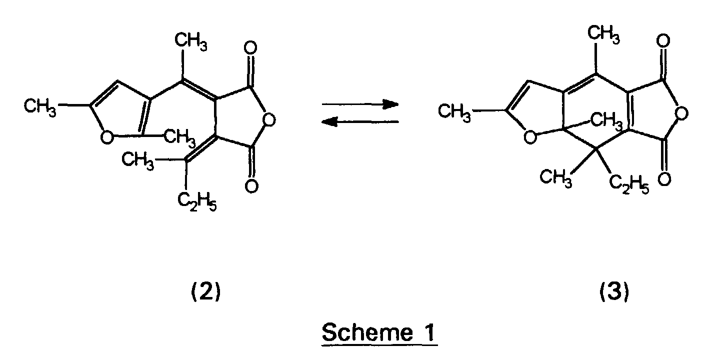

The cyclic forms of the fulgides, for example (3), are highly coloured, usually in the bright red to deep purple range and this is reported to arise from the extended, near planar, conjugated double bond structure with the oxygen heteroatom at one end of the molecule and the conjugated carbonyl at the other.

Compounds of formula (1) in which X is oxygen may be converted into the corresponding fulgimide derivatives (1, X = NR⁶) by reaction of the anhydride with a primary amine followed by an acid chloride in a suitable solvent such as dichloromethane.

As with the fulgides, fulgimides undergo photochemical ring closure to form the more highly coloured, thermally stable product. They exhibit photochromic properties similar to the corresponding fulgides, except that the long wavelength (visible) absorption bands of the coloured forms are generally broader and show bathochromic shifts. Furthermore fulgimides display greater resistance to hydrolysis compared with fulgides. Suitable fulgimide derivatives may be coupled with target biological systems, thereby giving such a system photochromic properties. For example, a fulgimide derivative in which R⁶ = CH₂COOEt was coupled via its N-hydroxysuccinimide ester to the nucleotide analogue, aminoallyl-2'-deoxyuridine 5'-triphosphate (V.Kiruvanayagam, PhD Thesis, University of Wales, (1995), p137).

Replacement of one or the other of the carbonyl groups of the fulgide structure with a substituted nitrogen results in two alternative isomers of fulgimides, termed α-isofulgimides (4) and β-isofulgimides (5). The compounds undergo photochemical ring closure to give coloured forms. 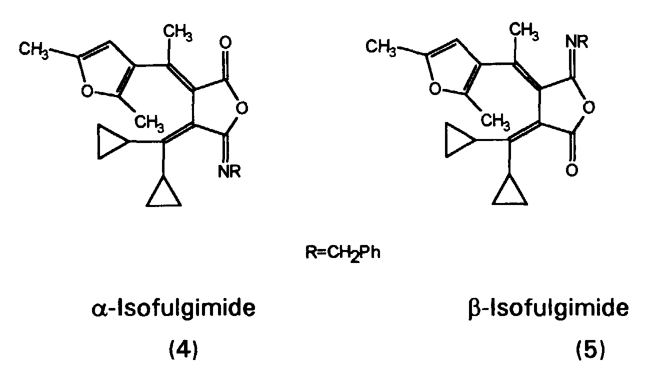 α-Isofulgimides may be prepared by the reaction of the appropriate succinic half-ester derivative with the Grignard derivative of the required amine, followed by cyclisation with DCC. β-Isofulgimides can be prepared by a similar method, but a more convenient approach is to derive the required succinamic acid from the corresponding fulgide, followed by reaction with DCC (K.S.V.Koh, PhD Thesis, University of Wales).

Accordingly the present invention provides compounds of formula (6); 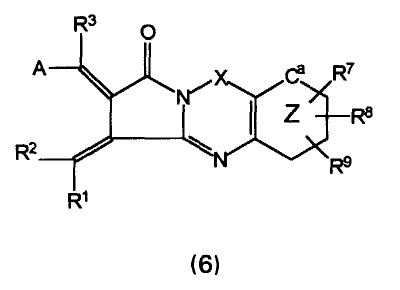 or a stereoisomer thereof, wherein groups R⁷, R⁸ and R⁹ are hydrogen or are chosen to provide desired solubility, reactivity and spectral properties to the compound;
X is a group -(CH₂)ₙ- or a group, 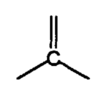 in which case it links via a two or three atom chain with carbon atom C^{a} of ring Z to form a fused bicyclic aromatic ring which may be optionally substituted and n is 0 or 1;
Z is an optionally substituted six-membered aromatic or fused bicyclic aromatic ring containing carbon atoms and optionally no more than two atoms selected from oxygen, nitrogen and sulphur;
R¹ and R² independently represent an alkyl, cycloalkyl, aryl, or an aralkyl group or one of R¹ and R² represents hydrogen and the other an alkyl, cycloalkyl, aryl, or an aralkyl group, or the group: 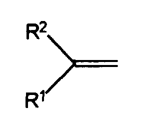 represents an adamantylidene group;
R³ represents hydrogen, alkyl, or aryl;
A represents a substituted or unsubstituted heterocyclic ring having one of the following structures: 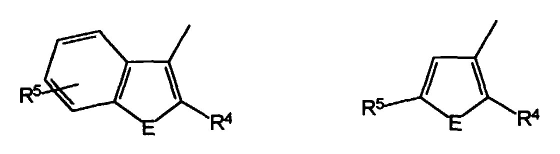 where R⁴ is selected from hydrogen, alkyl, aryl, and aralkyl groups, and R⁵ is selected from hydrogen, C₁₋₁₂ hydrocarbyl optionally substituted with halogen, C₁₋₆ alkoxy, aryl and C₆₋₁₂ aryloxy groups and E is selected from O, S and NR⁶, where R⁶ is selected from hydrogen, C₁₋₆ alkyl, or aralkyl.

Suitably, the two or three atom chain which links group X with carbon atom C^{a} of ring Z contains carbon atoms and optionally no more than one nitrogen atom. Preferably the two or three atom chain contains carbon atoms.

Optional substituents R⁷, R⁸ and R⁹ on the aromatic ring Z or the bicyclic aromatic ring that incorporates Z are the same or different and are independently selected from -R¹⁰ and -L-R¹⁰, wherein R¹⁰ is selected from: neutral groups that reduce water solubility; polar groups that increase water solubility; target bonding groups such as functional groups that can be used in labelling reactions; reactive groups; electron donating and withdrawing groups that shift the emission wavelengths of the photochromic molecule; lipid and hydrocarbon solubilising groups, and L is selected from the group consisting of a straight or branched C₁₋₁₀ alkyl chain, and a C₂₋₁₀ monoether or polyether.

Preferred R¹⁰ groups are selected from: hydrogen, halogen, amide, C₁-C₆ alkoxy, cyano, aryl, heteroaryl, sulphonate, quaternary ammonium, hydroxyl, optionally substituted amino, sulphydryl, carbonyl, and reactive groups, for example, succinimidyl ester and anhydride, and groups reactive with amino, hydroxyl, carboxyl, aldehyde, or sulphydryl groups.

Specific examples of the reactive groups R⁷, R⁸ and R⁹ and the groups with which R⁷, R⁸ and R⁹ will react are provided in Table 1. In the alternative, the R⁷, R⁸ and R⁹ may be the functional groups of Table 1 which would react with the reactive groups of a target molecule.

**Table 1**

| Possible Reactive Substituents and Functional Groups Reactive Therewith | |
|---|---|
| **Reactive Groups** | **Functional Groups** |
| succinimidyl esters | primary amino, secondary amino |
| anhydrides | primary amino, secondary amino, hydroxyl |
| substituted hydrazines, | aldehydes, ketones |
| acid halides | amino groups |
| haloacetamides, maleimides | thiols, imidazoles, hydroxyl, amine |
| carbodiimides | carboxyl groups |
| phosphoramidites | hydroxyl groups |

Preferred reactive groups R⁷, R⁸ and R⁹ which are especially useful for labelling target components with available amino and hydroxyl functional groups include: 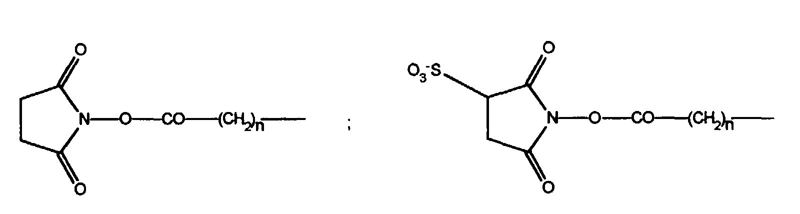 where n is 0 or an integer from 1-10.

Alkyl is a straight or branched chain alkyl group containing from 1-20 carbon atoms, preferably 1 to 6 carbon atoms, for example methyl, ethyl, n-propyl, iso-propyl and butyl.

Aryl is an aromatic substituent containing one or two fused aromatic rings containing 6 to 10 carbon atoms, for example phenyl or naphthyl, the aryl being optionally and independently substituted by one or more substituents, for example halogen, straight or branched chain alkyl groups containing 1 to 10 carbon atoms, cycloalkyl, aralkyl and alkoxy for example methoxy, ethoxy, propoxy and n-butoxy.

Cycloalkyl is a alicyclic substituent containing from 3 to 6 carbon atoms being attached by a single bond, for example cyclopropyl, cyclobutyl, cyclopentyl and cyclohexyl.

Heteroaryl is a mono- or bicyclic 5 to 10 membered aromatic ring system containing at least one and no more than 3 heteroatoms which may be selected from N, O, and S and is optionally and independently substituted by one or more substituents, for example halogen, straight or branched chain alkyl groups containing 1 to 20 carbon atoms, cycloalkyl, aralkyl and alkoxy for example methoxy, ethoxy, propoxy and n-butoxy.

Aralkyl is a C₁ to C₆ alkyl group substituted by an aryl or heteroaryl group.

Halogen and halo groups are selected from chlorine, bromine and iodine.

For the purpose of increasing water solubility or reducing unwanted non-specific binding of the photochromic compounds of the present invention to inappropriate components of a sample, one or more of the R⁷, R⁸ and R⁹ groups may be selected from well known polar or electrically charged chemical groups. Examples of such groups are -S-F, where F is hydroxy, sulphonate, carboxylate, substituted amino or quaternary amino and where S is a spacer group such as -(CH₂)ₙ- where n is 0 to 6. Examples of -S-F groups include C₁₋₆ alkyl sulphonates, such as -(CH₂)₃-SO₃⁻ and -(CH₂)₄-SO₃⁻.

Specific examples of the compounds of the present invention are as follows:
i) E-4-Dicyclopropylmethylene-3-[1-(2,5-dimethyl-3-furyl)ethylidene]-benzimidazol[1,2-a]pyrrolidin-2-one;
ii) E-4-Diphenylmethylene-3-[1-(2,5-dimethyl-3-thienyl)ethylidene]-benzimidazol[1,2-a]pyrrolidin-2-one;
iii) E-4-Dicyclopropylmethylene-7,8-dimethyl-3-[1-(2,5-dimethyl-5-phenyl-3-thienyl)ethylidene]benzimidazol[1,2-a]pyrrolidin-2-one;
iv) E-3-Adamantylidene-7,8-dimethyl-4-[1-(2,5-dimethyl-3-furyl)ethylidene]-benzimidazol[1,2-a]pyrrolidin-2-one;
v) E-3-Adamantylidene-4-[1-(2,5-dimethyl-3-furyl)ethylidene]-benzimidazol[1,2-a]pyrrolidin-2-one-7-carboxylic acid;
vi) E-7-Amino-4-dicyclopropylmethylene-3-[1-(2,5-dimethyl-3-furyl)ethylidene]benzimidazol[1,2-a]pyrrolidin-2-one.

The groups provided herein are not intended to be all-inclusive of those groups which can be incorporated at the R⁷, R⁸ and R⁹ sites of the present invention. It will be understood that there are various other groups which will react with groups on material that is to be labelled by the compounds of the present invention. Compounds produced by the incorporation of other such groups at the R⁷, R⁸ and R⁹ sites are intended to be encompassed by the present invention.

The compounds of the present invention may be used in one or more biological and non-biological applications. With respect to non-biological applications, compounds of the present invention having one or more uncharged groups at the R⁷, R⁸ and R⁹ positions, for example alkyl and aryl moieties, may be dissolved in non-polar materials to provide photochromic properties to those materials. Such non-polar materials include, for example paints, polymers, plastics, waxes, oils, inks and hydrocarbon solvents. Another non-biological application of the photochromic compounds of the present invention is to dissolve compounds having one or more charged and/or polar groups at the R⁷, R⁸ and R⁹ positions in polar solvents such as water, alcohols such as methanol or ethanol, ethylene glycol, or mixtures of such solvents.

Alternatively, the photochromic compounds of the present invention may contain a polymerizable group suitable for the formation of a polymer containing the complex. Suitable polymerizable groups are selected from acrylate, methacrylate and acrylamide. Polymerization may be carried out with a suitably derivatized compound of this invention used in conjunction with a second polymerizable monomer starting material, such as styrene or vinyltoluene, to form a copolymer containing the photochromic compound. The photochromic compounds need not have a polymerisable group, for example, the complex may be incorporated during polymerisation or particle formation or may be absorbed into or onto polymer particles.

Compounds of the present invention having a functional or reactive group at the R⁷, R⁸ and R⁹ positions may be used to covalently label a target material to impart photochromic properties to the target material, such as a carrier material, a luminescent compound or a biological material. The target bonding group may be a reactive group for reacting with a functional group on the target material. Alternatively the target bonding group may be a functional group for reaction with a reactive group on the target.

Suitable target materials may include a luminescent compound, such as fluorescent dyes (based for example on the fluorescein, rhodamine, coumarin, pyrene and cyanine chromophores), antibodies, antigens, proteins, carbohydrates, lipids, nucleotides which contain or are derivatized to contain one of amino, hydroxyl, sulphydryl, carboxyl, or carbonyl groups, and oxy or deoxy polynucleic acids which contain or are derivatized to contain one of amino, hydroxyl, phosphate, thiophosphoryl, sulphydryl, carboxyl, or carbonyl groups, cells, polymer particles, or glass beads. Covalent labelling using compounds of the present invention may be accomplished with a target having at least one functional or reactive group as defined hereinbefore. The target may be reacted with an amount of a compound of the present invention having at least one of R⁸ to R⁹ that includes a reactive or functional group as hereinbefore defined that can covalently bind with the functional or reactive group of the target material. The target material and the compound of the present invention are incubated under conditions and for a period of time sufficient to permit the target material to covalently bond to the compound of the present invention.

The present invention also provides a process for the preparation of a compound of formula (6) which comprises reaction of a compound of formula (7), 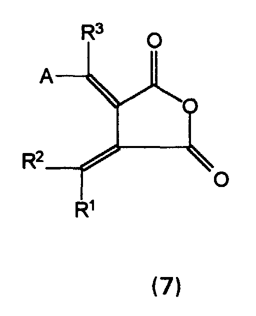 or its corresponding di-carboxylic acid, di-C₁ - C₆ alkyl ester, or mono-carboxylic acid-mono C₁ - C₆ alkyl ester derivative, wherein R¹, R², R³ and A are as hereinbefore defined with a compound of formula (8): 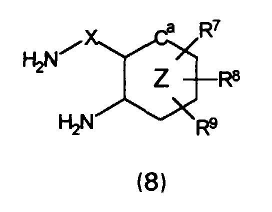 or a salt thereof, optionally substituted by groups R⁷, R⁸ and R⁹, wherein R⁷, R⁸ and R⁹, X and Z are as hereinbefore defined. The reaction is suitably carried out in a dry, inert solvent such as toluene and in the absence of light. The reaction is suitably carried out at an elevated temperature, for example 50°C to 150°C, suitably 100°C to 125°C. The reaction mixture (containing a mixture of geometric isomers of the product) is fractionated into separate isomers by column chromatography using silica gel, or by fractional crystallisation.

The Stobbe condensation provides a general method for preparing compounds of the general formula (7). An account of this reaction and its application to the synthesis of a wide range of succinic acid derivatives is given in Organic Reactions, Vol. 6, pp1-73, published by Wiley, New York 1951. For example, compounds of formula (7) can be prepared by reaction of a compound of formula (9), 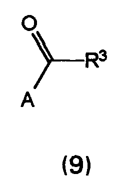 wherein R³ and A are as hereinbefore defined with a succinic ester of formula (10), 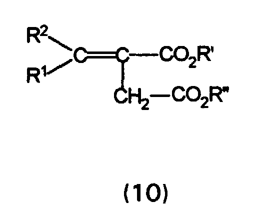 where R¹ and R² are as hereinbefore defined and R' and R'' are independently selected from methyl, ethyl, n-propyl, and n-butyl, by a Stobbe condensation to yield a product of formula (7). Preferably the Stobbe condensation may be carried out by treating the reactants in t-butanol, or toluene, or tetrahydrofuran containing potassium t-butoxide. The product at this stage is the half-ester, ie where one of the R' or R'' groups is hydrogen. The half-ester is then converted into the di-acid by hydrolysis, for example by boiling with ethanolic potassium hydroxide. The di-acid is then converted into its anhydride by a dehydration reaction, comprising stirring at ambient temperature with an acid chloride. Preferably acetyl chloride is used for this purpose.

For example:
(i) Compounds of formula (7) can be prepared using as the starting material a ketone of formula (11), 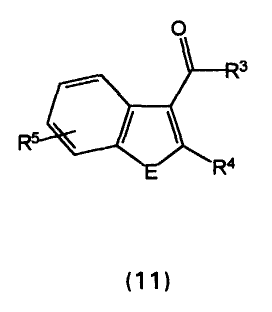 wherein R³, R⁴, R⁵ and E are as hereinbefore defined with a succinic ester of formula (10), where R¹, R², R' and R'' are hereinbefore defined.
(ii) Compounds of formula (7) above can also be prepared in an analogous manner to that described above using adamantan-2-one as a starting material. Preparation of adamantan-2-one is described in US Patent No. 3257456 and by Gulak et al, Organic Synthesis, 53, 8, (1973). Compounds of formula (7) can be prepared by refluxing adamantan-2-one with a succinate diester in a solution of potassium t-butoxide in t-butanol to give the potassium salt of the corresponding half ester, which is converted into an adamant-2-ylidene succinate diester of formula (12), in which R' and R'' are hereinbefore defined. 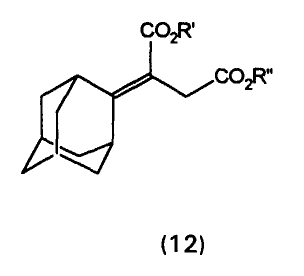 Compounds of formula (7) containing an adamantylidene group are obtained by reacting a diester of formula (12) with a ketone of formula (9) or of formula (11) in the presence of sodium hydride, in a dry inert solvent such as toluene.

See for example, US Patent No.4220708, the disclosure of which is incorporated by reference.

Precursor compounds of formula (8) are readily available or may be prepared by methods well known to those skilled in the art.

It will be readily appreciated that certain compounds of formula (6) may be useful as intermediates for conversion to other compounds of the formula (6) by methods well known to those skilled in the art. Likewise, certain of the intermediates may be useful for the synthesis of derivatives of formula (6). The compounds of the present invention may be synthesized by the methods disclosed herein. Derivatives of the compounds having a particular utility are prepared either by selecting appropriate precursors or by modifying the resultant compounds by known methods to include functional groups at a variety of positions. As examples, the compounds of the present invention may be modified to include certain reactive groups for preparing a photochromic labelling reagent, or charged or polar groups may be added to enhance the solubility of the compound in polar or nonpolar solvents or materials. As examples of conversions, carboxylic acid groups may be converted into esters, and amide groups.

Compounds of the present invention switch from near colourless to coloured forms on exposure to UV light and the colour of the coloured form is pH dependent. The following are specific examples of the synthesis of compounds of the present invention.

### Experimental Section

### i) General

Ultraviolet spectra were recorded on a Cecil CE6600 spectrophotometer and a Perkin Elmer Lambda 20UV/Vis spectrophotometer for 1 x 10⁻⁴M solutions in dry toluene.

¹H NMR spectra were obtained using a Bruker WM 360 (360MHz) FTNMR spectrometer for solutions in deuterated chloroform with 1% TMS as internal standard. Microanalyses were obtained using a Perkin Elmer 240B analyser. Melting points were measured on a Reichert Hot Stage Microscope.

### ii) General Synthesis of Compounds of Formula (6)

A solution of a compound of formula (7) and a compound of formula (8) in toluene was boiled (24 hours) in the absence of light. Solvent was removed under reduced pressure and the residual oil was purified by column chromatography on silica gel, using usually mixtures of diethyl ether and petroleum ether (40-60°C) as eluant. The main fractions gave compounds of formula (6) usually as yellow crystals after recrystallisation from diethyl ether and petroleum ether (40-60°C).

### Example 1: Preparation of 1a, 1b and 1c

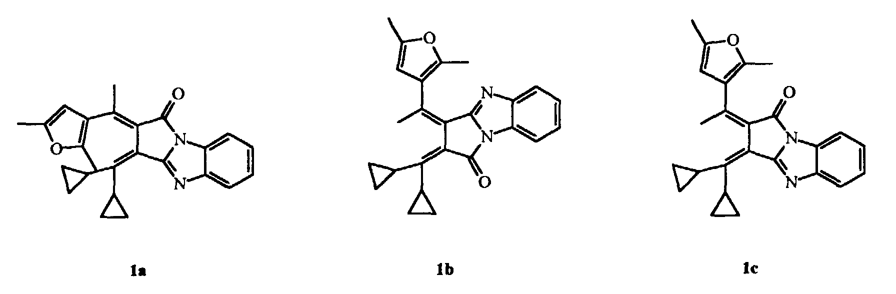

A solution of E/Z-3-dicyclopropylmethylene-4-[1-(2,5-dimethyl-3-furyl)ethylidene]succinic anhydride (5.00g, 16.03mmoles) and 1,2-phenylenediamine (2.08g, 19.23mmoles) in toluene (50ml) was boiled for 23 hours. Removal of solvent left an oil consisting of a mixture of compounds of formula 1a, 1b and 1c, which were separated and purified by column chromatography and by fractional recrystallisation from diethyl ether and petroleum ether (40-60°C).

### 1a: E-4-Dicyclopropylmethylene-3-[1-(2,5-dimethyl-3-furyl)ethylidene]-benzimidazol[1,2-a]pyrrolidin-2-one

Yellow crystals (0.808g, 13.13%), m.p. 148-149°C, m/z 384.2. Found: C, 78.15; H, 6.22; N, 7.19%. C₂₅H₂₄N₂O₂ requires C, 78.10; H, 6.25; N, 7.29%.

### 1b: Z--3-Dicyclopropylmethylene-4-[1 -(2,5-dimethyl-3-furyl)ethylidene]-benzimidazol[1,2-a]pyrrolidin-2-one

Yellow crystals (0.649g, 10.55%), m.p. 192-194°C, m/z 384.2. Found: C, 77.92; H, 6.44; N, 7.13%. C₂₅H₂₄N₂O₂ requires C, 78.10; H, 6.25; N, 7.29%.

### 1c: Z--4-Dicyclopropylmethylene-3-[1-(2,5-dimethyl-3-furyl)ethylidene]-benzimidazol[1,2-a]pyrrolidin-2-one

Yellow crystals (0.251g, 4.08%), m.p. 180-183°C, m/z 384.2. Found: C, 78.05; H, 6.37; N, 7.04%. C₂₅H₂₄N₂O₂ requires C, 78.10; H, 6.25; N, 7.29%.

### Example 2: Preparation of 2a and 2b

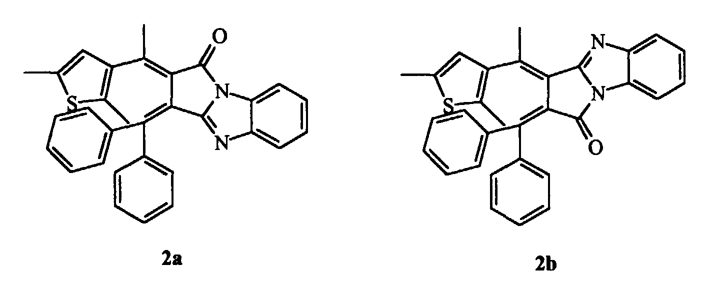

A solution of E-[1-(2,5-dimethyl-3-thienyl)ethylideneldiphenylmethylenesuccinic anhydride (0.50g, 1.25mmoles) and 1,2-phenylenediamine (0.16g, 1.48mmoles) in toluene (30ml) was boiled for 68 hours. Removal of solvent left an oil consisting of a mixture of compounds of formula 2a and 2b, which were separated and purified by column chromatography and by fractional recrystallisation from diethyl ether and petroleum ether (40-60°C).

### 2a: E-4-Diphenylmethylene-3-[1-(2,5-dimethyl-3-thienyl)ethylidene]-benzimidazol[1,2-a]pyrrolidin-2-one

Yellow powder (0.055g, 9,34%), m.p. 217-223.5°C, m/z 472.1. Found: C, 78.78; H, 5.32; N, 6.01%. C₃₁H₂₄N₂OS requires C, 78.79; H, 5.12; N, 5.93%.

### 2b: E-3-Diphenylmethylene-4-[1-(2,5-dimethyl-3-thienyl)ethylidene]-benzimidazol[1,2-a]pyrrolidin-2-one

Yellow powder (0.194g, 32.93%), m.p. 224-227°C, m/z 472.1. Found: C, 78.99; H, 5.32; N, 5.91%. C₃₁H₂₄N₂OS requires C, 78.79; H, 5.12; N, 5.93%.

### Example 3: Preparation of 3a and 3b

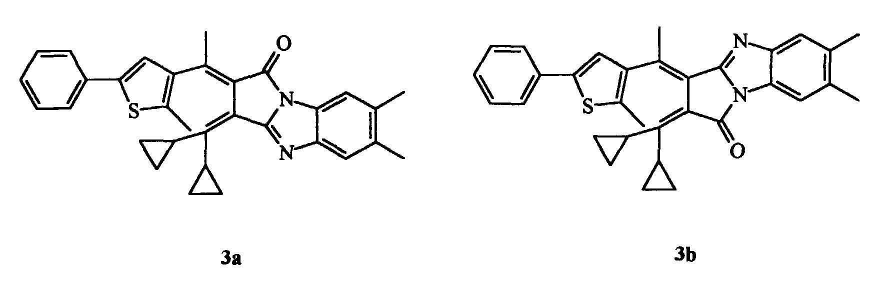

A solution of E-3-dicyclopropylmethylene-4-[1-(2-methyl-5-phenyl-3-thienyl)ethylidene] succinic anhydride (1.00g, 2.56mmoles) and 4,5-dimethyl-1,2-phenylenediamine (0.42g, 3.08mmoles) in toluene (40ml) was boiled for 69 hours. Removal of solvent left an oil consisting of a mixture of compounds of formula 3a and 3b, which were separated and purified by column chromatography and by fractional recrystallisation in diethyl ether and petroleum ether (40-60°C).

### 3a: E-4-Dicyclopropylmethylene-7,8-dimethyl-3-[1-(2,5-dimethyl-5-phenyl-3-thienyl)ethylidene]benzimidazol[1,2-a]pyrrolidin-2-one

Green/yellow crystals (0.161g, 12.81%), m.p. 213.5-215.5°C, m/z 490.0. Found: C, 78.30; H, 6.20; N, 5.65%. C₃₂H₃₀N₂OS requires C, 78.33; H, 6.16; N, 5.71%.

### 3b: E-3-Dicyclopropylmethylene-7,8-dimethyl-4-[1-(2,5-dimethyl-5-phenyl-3-thienyl)ethylidene]benzimidazol[1,2-a]pyrrolidin-2-one

Pale yellow crystals (0.275g, 21.90%), m.p. 233-236°C, m/z 490.1. Found: C, 78.12; H, 6.34; N, 5.78%. C₃₂H₃₀N₂OS requires C, 78.33; H, 6.16; N, 5.71 %.

### Example 4: Preparation of 4a and 4b

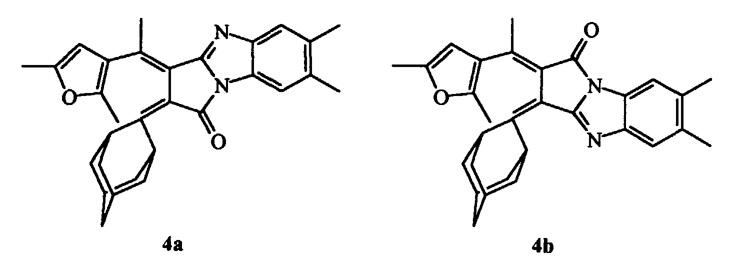

A solution of E-3-adamantylidene-4-[1-(2,5-dimethyl-3-furyl)ethylidene}succinic anhydride (1.00g, 2.84mmoles) and 4,5-dimethyl-1,2-phenylenediamine (0.58g, 4.26mmoles) in toluene (40ml) was boiled for 22 hours. Removal of solvent left an oil consisting of a mixture of compounds of formula 4a and 4b, which were separated and purified by column chromatography and by fractional recrystallisation in diethyl ether and petroleum ether (40-60°C).

### 4a: E-3-Adamantylidene-7,8-dimethyl-4-[1-(2,5-dimethyl-3-furyl)ethylidene]-benzimidazol[1,2-a]pyrrolidin-2-one

Yellow/Green cubed shaped crystals (0.261g, 20.29%), m.p. 231-234°C, m/z 452.3. Found: C, 79.84; H, 6.91; N, 6.09%. C₃₀H₃₂N₂O₂ requires C, 79.61; H, 7.13; N, 6.19%.

### 4b: E-4-Adamantylidene-7,8-dimethyl-3-[1-(2,5-dimethyl-3-furyl)ethylidene]-benzimidazol[1,2-a]pyrrolidin-2-one

Blue crystals (0.079g, 6.15%), m.p. 235.5-237.5°C, m/z 452.3. Found: C, 79.35; H, 7.25; N, 6.24%. C₃₀H₃₂N₂O₂ requires C, 79.61; H, 7.13; N, 6.19%.

### Example 5: Preparation of 5a

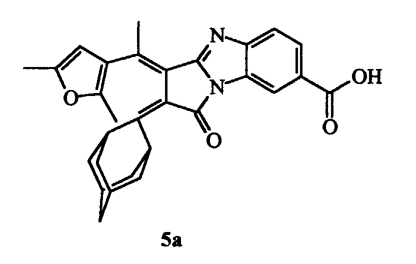

A solution E-3-adamantyIidene-4-[1-(2,5-dimethyl-3-furyl)ethylidene}succinic anhydride (1.00g, 2.84mmoles), 3,4-diaminobenzoic acid (0.52, 3.42mmoles) and a catalytic amount of potassium tert butoxide (0.032g, 2.85x10⁻⁴moles) in toluene (30ml) were boiled for 49 hours. Removal of solvent left an oil consisting of compound of formula 5a, which was separated and purified by column chromatography and by fractional recrystallisation in a hot 50%:50% toluene/ethanol solution.

### 5a: E-3-Adamantylidene-4-[1-(2,5-dimethyl-3-furyl)ethylidene]benzimidazol-[1,2-a]pyrrolidin-2-one-7-carboxylic acid

Pale yellow spherical clusters (0.840g, 63.18%), m.p. 264-267°C, m/z 468.2. Found: C, 74.12; H, 6.15; N, 5.68%. C₂₉H₂₈N₂O₄ requires C, 74.34; H, 6.02; N, 5.98%.

### Example 6: Preparation of 6a and 6b

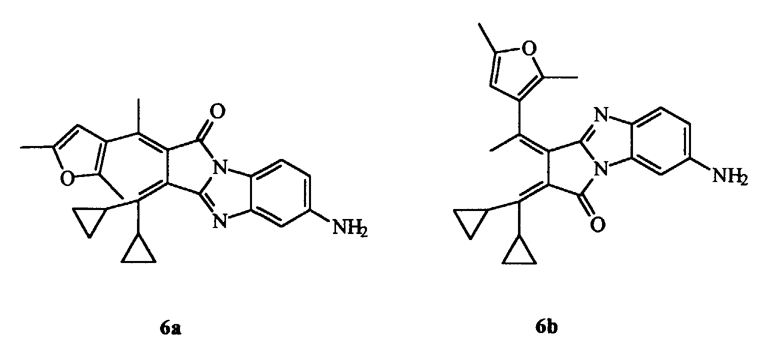

A solution of E/Z-3-dicyclopropylmethylene-4-[1-(2,5-dimethyl-3-furyl)ethylidene]succinic anhydride (1.50g, 4.81mmoles) and 4-amino-1,2-phenylenediamine, [made from the catalytic hydrogenation of 4-nitro-1,2-phenylenediamine (2.00g, 13.07mmoles) using Raney nickel and hydrazine (2ml)], in toluene (40ml) was boiled for 96 hours. Removal of solvent left an oil consisting of compounds of formula **6a** and **6b**, which were separated and purified by column chromatography and by fractional recrystallisation in diethyl ether and petroleum ether (40-60°C).

### 6a: E-7-Amino-4-dicyclopropylmethylene-3-[1-(2,5-dimethyl-3-furyl)ethylidene]benzimidazol[1,2-a]pyrrolidin-2-one

Yellow/brown powder (0.106g, 5.53%), m.p. 91-94°C, m/z 399.4. Found: C, 74.96; H, 6.54; N, 10.71%. C₂₅H₂₅N₃O₂ requires C, 75.19; H, 6.27; N, 10.53%.

### 6b Z-7-Amino-3-dicyclopropylmethylene-4-[1-(2,5-dimethyl-3-furyl)ethylidene]benzimidazol[1,2-a]pyrrolidin-2-one

Green powder (0.133g, 6.93%), m.p. 78-80°C, m/z 399.5. Found: C, 74.92; H, 6.52; N, 10.62%. C₂₅H₂₅N₃O₂ requires C, 75.19; H, 6.27; N, 10.53%.

## Claims

1. A compound of formula: 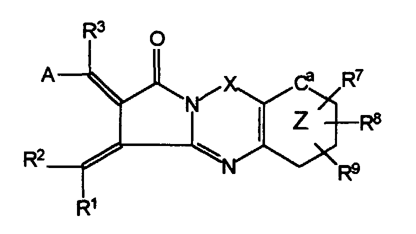 or a stereoisomer thereof, wherein groups R⁷, R⁸ and R⁹ are hydrogen or are chosen to provide desired solubility, reactivity and spectral properties to the compound;
X is a group -(CH₂)ₙ- or a group, 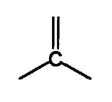 in which case it links via a two or three atom chain with carbon atom C^{a} of ring Z to form a fused bicyclic aromatic ring which may be optionally substituted and n is 0 or 1;
Z is an optionally substituted six-membered aromatic or fused bicyclic aromatic ring containing carbon atoms and optionally no more than two atoms selected from oxygen, nitrogen and sulphur;
R¹ and R² independently represent an alkyl, cycloalkyl, aryl, or an aralkyl group or one of R¹ and R² represents hydrogen and the other an alkyl, cycloalkyl, aryl, or an aralkyl group, or the group: 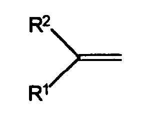 represents an adamantylidene group;
R³ represents hydrogen, alkyl, or aryl;
A represents a substituted or unsubstituted heterocyclic ring having one of the following structures: 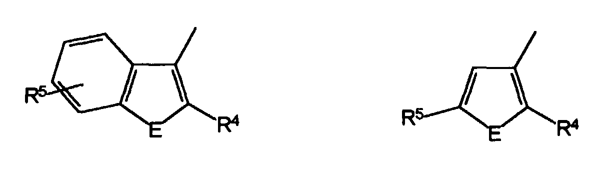 where R⁴ is selected from hydrogen, alkyl, aryl, and aralkyl groups, and R⁵ is selected from hydrogen, C₁₋₁₂ hydrocarbyl optionally substituted with halogen, C₁₋₆ alkoxy, aryl, and C₆₋₁₂ aryloxy groups and E is selected from O, S and NR⁶, where R⁶ is selected from hydrogen, C₁₋₆ alkyl, or aralkyl.

2. A compound according to Claim 1 wherein R¹ and R² each independently represent lower alkyl groups including cycloalkyl groups having 1-6 carbon atoms.

3. A compound according to Claim 1 wherein R³ represents a lower alkyl group having 1-6 carbon atoms.

4. A compound according to any of Claims 1-3 wherein R⁴ represents a lower alkyl group having 1-6 carbon atoms, phenyl or a substituted phenyl group.

5. A compound according to any of Claims 1-4 wherein R⁷, R⁸ and R⁹ are the same or different and are selected from -R¹⁰ and -L-R¹⁰ wherein R¹⁰ is selected from: neutral groups that reduce water solubility; polar groups that increase water solubility; target bonding groups such as functional groups that can be used in labelling reactions; reactive groups; electron donating and withdrawing groups that shift the absorption and emission wavelengths of the photochromic molecule; lipid and hydrocarbon solubilising groups; and L is selected from the group consisting of a straight or branched C₁₋₁₀ alkyl chain, and a C₂₋₁₀ monoether or polyether.

6. A compound according to any of Claims 1-5 wherein R¹⁰ is selected from: hydrogen, halogen, amide, C₁-C₆ alkoxy, cyano, aryl, heteroaryl, sulphonate, quaternary ammonium, hydroxyl, optionally substituted amino, sulphydryl, carbonyl, and reactive groups, for example, succinimidyl ester, anhydride, haloacetamide, maleimide, phosphoramidite, hydrazide and carbodiimide; and groups reactive with amino, hydroxyl, carboxyl, aldehyde, or sulphydryl groups.

7. A compound according to Claim 1 selected from:
i) E-4-Dicyclopropylmethylene-3-[1-(2,5-dimethyl-3-furyl)ethylidene]-benzimidazol[1,2-a]pyrrolidin-2-one;
ii) E-4-Diphenylmethylene-3-[1-(2,5-dimethyl-3-thienyl)ethylidene]-benzimidazol[1,2-a]pyrrolidin-2-one;
iii) E-4-Dicyclopropylmethylene-7,8-dimethyl-3-[1-(2,5-dimethyl-5-phenyl-3-thienyl)ethylidene]benzimidazol[1,2-a]pyrrolidin-2-one;
iv) E-3-Adamantylidene-7,8-dimethyl-4-[1-(2,5-dimethyl-3-furyl)ethylidene]-benzimidazol[1,2-a]pyrrolidin-2-one;
v) E-3-Adamantylidene-4-[1-(2,5-dimethyl-3-furyl)ethylidene]-benzimidazol[1,2-a]pyrrolidin-2-one-7-carboxylic acid;
vi) E-7-Amino-4-dicyclopropylmethylene-3-[1-(2,5-dimethyl-3-furyl)ethylidene]benzimidazol[1,2-a]pyrrolidin-2-one.

8. A method for producing a compound according to any of Claims 1-7 comprising reacting a compound of formula: 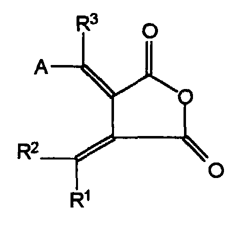 or its corresponding di-carboxylic acid, di-C₁ - C₆ alkyl ester, or mono-carboxylic acid-mono C₁ - C₆ alkyl ester derivative, wherein R¹, R², R³ and A are as hereinbefore defined with a compound of formula: 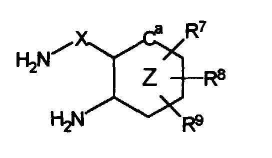 or a salt thereof, optionally substituted by groups R⁷, R⁸ and R⁹, wherein R⁷, R⁸, R⁹, X and Z are as hereinbefore defined.

9. A method of imparting photochromic properties to a non-polar material, the method comprising the step of admixing the non-polar material with a compound as recited in any one of Claims 1-7, wherein at least one groups R⁷, R⁸ and R⁹ is an uncharged group.

10. A method of imparting photochromic properties to a polar material, the method comprising the step of admixing the polar material with a compound as claimed in any one of claims 1-7 wherein at least one of the groups R⁷, R⁸ and R⁹ is selected from the group consisting of charged groups and polar groups.

11. A method for imparting photochromic properties to a target material, the method comprising the steps of incubating:
i) a target material having at least one functional group selected from the group consisting of amino, hydroxyl, carbonyl and sulphydryl groups; or having at least one reactive group that can covalently bond with said at least one functional group, and;
ii) an amount of the photochromic compound as claimed in any one of claims 1-7 wherein at least one of groups R⁷, R⁸ and R⁹ is a functional group selected from the group consisting of amino, hydroxyl, carbonyl and sulphydryl; or wherein at least one of groups R⁷, R⁸ and R⁹ is a reactive group that can covalently bond with said at least one functional group;
for a period of time sufficient to permit said at least one functional or reactive group of said fluorescent compound to covalently bond to said at least one reactive or functional group of said target material.

12. A target material covalently labeled with a compound according to any one of claims 1-7.
